# FASCICULE DE BREVET EUROPEEN

(11) **EP 3 215 826 B1**
(45) Date de publication et mention de la délivrance du brevet: **26.08.2020**
(21) Numéro de dépôt: 15805579.8
(22) Date de dépôt: 05.11.2015
(51) Int. Cl.: G01N 19/04, G01M 5/00

(54) **ÉPROUVETTE DE TESTS EN PELAGE**
ABREISSTESTABSCHNITT
PEELING TEST COUPON

(30) Priorité: 06.11.2014 FR 1460740
(43) Date de publication de la demande: 13.09.2017
(73) Titulaire: SAFRAN AIRCRAFT ENGINES, 75015 Paris (FR)
(72) Inventeur: BROSSIER, Pascal, Noël, 77550 Moissy Cramayel (FR); BERRANGER, Thibault, 77550 Moissy Cramayel (FR); CHICHERY, Emmanuel, 77550 Moissy Cramayel (FR); GANI, Léa, 77550 Moissy Cramayel (FR); TIMON, Alain, 77550 Moissy Cramayel (FR)
(74) Mandataire: Ernest Gutmann - Yves Plasseraud S.A.S.
(86) Numéro de dépôt international: PCT/FR2015/053000
(87) Numéro de publication internationale: WO 2016/071647

(56) Documents cités:
- EP-A2- 2 549 263
- DE-U1- 9 013 935
- DE-U1- 9 017 803
- US-A- 5 317 925
- US-A- 5 626 462
- US-B1- 7 028 555
- US-B1- 8 215 181

## Description

L'invention se rapporte à une éprouvette de tests en pelage. Par ailleurs, l'invention concerne un procédé de fabrication de telles éprouvettes.

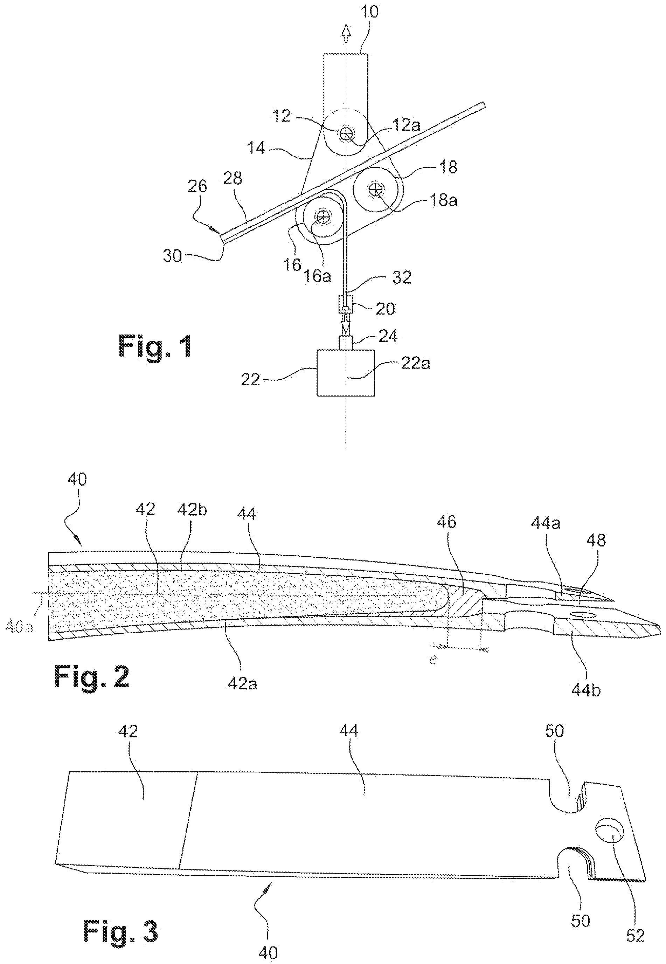

On décrit ci-après, en référence à la figure 1, un dispositif de tests en pelage comprenant son éprouvette de test, déjà connu, conformément aux préconisations énoncées par la norme NF EN 1464.

Ainsi, selon l'état connu de la technique, un dispositif de tests en pelage comprend un bâti 10 auquel est suspendu autour d'une liaison pivot 12 d'axe horizontal 12a un pendule 14 comprenant un rouleau de front 16 et un rouleau de guidage 18, d'axes respectifs 16a et 18a parallèles à l'axe 12a. L'angle entre les directions (16a ; 18a) et (18a ; 12a) est de 90°. Les distances [16a ; 18a] et [18a ; 12a] valent chacune 33 mm.

Des moyens d'accrochage 20 sont disposés sous le pendule 14 et sont reliés à des moyens de traction 22 configurés pour tracter les moyens d'accrochage 20 vers le bas verticalement, selon une direction 22a passant par l'axe pivot 12a. Entre les moyens d'accrochage 20 et les moyens de traction 22 sont intercalés un capteur de force 24 pouvant mesurer la force de traction mise en oeuvre entre les moyens de traction 22 et les moyens d'accrochage 20, c'est-à-dire la force de traction vers le bas des moyens d'accrochage 20.

Une éprouvette 26 est utilisée. Celle-ci comprend un support 28 de forme plate et rectangulaire, et un adhésif 30 collé sur une surface du support 28. L'éprouvette est positionnée sur les deux rouleaux 16, 18 de front et de guidage dans sa longueur, l'adhésif 30 vers le bas en appui sur les rouleaux, et une partie d'extrémité 32 de l'adhésif 30 est pré-décollée et est rendue accessible entre les deux rouleaux 16, 18. Les moyens d'accrochage 20, ici des mors, sont accrochés par serrage à l'extrémité 32 de l'adhésif 30.

Pour lancer le test en pelage, on actionne les moyens de traction 22 avec une contrainte de vitesse de déplacement constante, et on relève les valeurs transmises par le capteur de force 24. Lorsque les moyens de traction 22 sont actionnés, l'extrémité 32 de l'adhésif 30 se déforme et est tendue vers le bas. L'adhésif 30 se décolle petit à petit du support 28, suivant un front de pelage, et est entrainé vers le bas tangentiellement au rouleau de front 16, alors que le support 28 se déplace en roulant du rouleau de front 16 au rouleau de guidage 18.

Lors du pelage, le pendule 14 bascule autour du pivot 12 jusqu'à ce que la surface du rouleau de front 16 soit tangente à la direction de traction 22a à la verticale de l'axe pivot 12a.

Bien que ce dispositif de test en pelage soit très bien adapté à des éprouvettes réalisées spécifiquement et de géométries simples, il ne convient en revanche pas à des éprouvettes de formes plus complexes, telles que celles présentant des courbures variables pour chacune de leurs faces, des épaisseurs variables, et des faces non parallèles. En effet, afin d'exploiter valablement la totalité des tests de pelage, l'angle de pelage, c'est-à-dire l'angle entre la surface du support où se trouve le front de pelage, et la direction de traction de l'adhésif, doit rester le même pour tous les tests en pelage effectués sur les différentes éprouvettes. Ceci n'est pas possible avec des éprouvettes complexes et variables comme celles évoquées ci-dessus.

A cet égard, US8215181 divulgue une éprouvette de tests en pelage. Malgré cela, un problème demeure lié à comment parvenir à réaliser un test le plus proche de la réalité correspondant typiquement à la collision d'un objet avec une aube.

Partant de cette constatation, l'invention apporte une solution simple, efficace et économique, permettant d'effectuer des tests en pelage respectant les normes définies, sur des éprouvettes de formes complexes.

A cet effet, elle propose une éprouvette de tests en pelage, avec pour caractéristiques que :
- l'éprouvette est issue d'une aube, et comprend :
   -- une portion de pale de l'aube qui comporte une surface d'intrados, une surface d'extrados, et un bord d'attaque et/ou un bord de fuite, et
   -- un renfort de l'aube qui recouvre et est collé sur au moins une partie de la surface d'intrados et une partie de la surface d'extrados, et qui s'étend au-delà de l'aube et du bord d'attaque et/ou du bord de fuite de la portion de pale,
- ledit renfort est fendu sur toute la longueur du bord d'attaque et/ou du bord de fuite, de manière à ce que le renfort soit séparé en deux plaques distinctes l'une de l'autre se faisant face de part et d'autre de la fente, au-delà du bord d'attaque et/ou du bord de fuite de la portion de pale, et
- au moins une des plaques comprend en outre, au-delà du bord d'attaque et/ou du bord de fuite de la portion de pale, des moyens d'attache procurant à cette même plaque une prise pour une traction.

La possibilité offerte par l'invention, de déplacer au moins un des rouleaux parmi ceux de front ou de guidage par rapport au bâti, permet de modifier le positionnement de l'éprouvette par rapport au bâti avant le commencement du test en pelage ou pendant le test en pelage.

Pour fonctionner, l'éprouvette doit être en appui par sa face comportant l'adhésif sur le rouleau de front. C'est tangentiellement à ce rouleau que l'adhésif est tracté en éloignement du support pour le décoller du support, selon un angle prédéfini par rapport à la surface du support appelé angle de pelage. Ainsi, lors du pelage, l'adhésif se décolle du support et longe le rouleau de front, alors que le support est entrainé en roulement sur les rouleaux. Le test en pelage tend à faire pivoter l'éprouvette autour du rouleau de front. C'est pourquoi les rouleaux de guidages sont fournis pour retenir l'éprouvette dans une orientation constante par rapport au bâti.

Dans un premier cas, l'éprouvette peut être retenue par les rouleaux de guidage par sa face comprenant l'adhésif à peler. Dans ce cas, au moins un rouleau de guidage doit être positionné en aval du front de pelage, c'est-à-dire du côté du front de pelage créé opposé au rouleau de front. L'éprouvette est alors posée sur les rouleaux de front et de guidage.

Dans un deuxième cas l'éprouvette peut être retenue par les rouleaux de guidage par sa face opposée à l'adhésif à peler. Dans ce cas, au moins un rouleau de guidage doit être positionné en amont du front de pelage, c'est-à-dire du même côté du front de pelage créé que le rouleau de front. L'éprouvette est alors logée entre le rouleau de front et les rouleaux de guidage.

Des éprouvettes de courbures respectives différentes, ou d'épaisseurs respectives différentes, en appuis sur des rouleaux non réglables par rapport au bâti, lors de la mise en tension des moyens de traction et de l'adhésif, auraient des orientations respectives différentes les unes des autres dans le plan perpendiculaire aux axes des rouleaux, lors de tests en pelage successifs. Les angles de pelage pour les différents tests seraient donc tous différents, et les tests ne seraient pas comparables les uns avec les autres. Au contraire, en modifiant la position de l'un des rouleaux comme indiqué par l'invention, on peut faire varier l'inclinaison de l'éprouvette. Ceci présente un intérêt évident puisque le réglage en translation des rouleaux permet d'obtenir une orientation des éprouvettes, tangentiellement au rouleau de front, toujours identique. Les différents tests en pelage effectués le sont donc tous selon le même angle de pelage, et sont donc comparables les uns aux autres.

De même, si la courbure de l'éprouvette est variable sur son étendue, on peut également faire varier la position de l'un des rouleaux lors du test en pelage, alors que l'éprouvette s'avance tangentiellement aux rouleaux, afin de conserver le même angle de pelage pendant toute la durée du test.

Selon une caractéristique de l'invention, les rouleaux de guidage possèdent chacun une surface locale annulaire d'appui externe d'aspect linéaire.

Cette caractéristique peut être atteinte par exemple lorsque la surface d'appui externe est d'allure ellipsoïdale, par exemple sphérique, ou encore torique. Grâce à cette caractéristique, le rouleau présente sur l'ensemble de son pourtour une surface d'appui permettant d'obtenir une liaison de type ponctuelle avec une surface plane en vis-à-vis.

Cette caractéristique est fonctionnelle et présente un avantage, comme il est expliqué par la suite, en combinaison avec et indépendamment de la caractéristique selon laquelle au moins un des rouleaux est réglable en translation par rapport aux autres rouleaux dans une direction perpendiculaire aux axes des rouleaux.

En liaison avec ce qui précède, on notera aussi qu'un avantage pourra être obtenu lorsqu'une éprouvette possède une face où est collé l'adhésif non parallèle à sa face opposée, et/ou torsadée sur son long. Pour de tels cas de figure, si un des rouleaux de guidage présentait une liaison linéaire avec l'éprouvette, en possédant une surface cylindrique ou tronconique par exemple, d'axe parallèle au rouleau de front, le rouleau de front pourrait ne pas assurer un appui stable sur une telle éprouvette. Au contraire, si l'on forme les rouleaux de guidage afin de réaliser une liaison ponctuelle quelle que soit la position angulaire du rouleau, la surface de l'éprouvette comportant l'adhésif pourra rester en appui complet sur le rouleau de front, alors que le reste de l'éprouvette pourra pivoter autour des contacts ponctuels des rouleaux de guidage.

Avantageusement, les moyens d'accrochage comprennent un câble destiné à s'engager autour de l'adhésif de l'éprouvette. Un tel câble peut être à la fois suffisamment fin, souple et résistant, par exemple en kevlar™, ce qui correspond tout à fait aux besoins de flexibilité et de solidité des moyens d'accrochage.

Préférentiellement, le rouleau de front est réglable en translation par rapport au bâti dans une direction parallèle au sens de traction, et un des rouleaux de guidage est réglable en translation par rapport au bâti dans une direction perpendiculaire au sens de traction.

Le rouleau de front est réglable en translation par rapport au bâti dans une direction parallèle au sens de traction afin que le rouleau de front puisse rester tangent au sens de traction même après son réglage, ce qui est nécessaire :
- au bon fonctionnement d'un dispositif de test en pelage qui utiliserait l'éprouvette qui a été présentées ci-avant, et
- au respect de la norme de pelage.

Partant de cette considération, le fait de permettre à un rouleau de guidage d'être réglable en translation dans une direction perpendiculaire au sens de traction permet de maximiser les possibilités d'orientation de l'éprouvette.

De manière plus précise, le sens de traction est fixe par rapport au bâti, et le rouleau de front est seulement réglable en translation par rapport au bâti dans une direction parallèle au sens de traction. Il est en effet plus sûr de proposer un sens de traction toujours fixe, car dans ce cas il n'y pas de risque que le sens de traction varie au cours des tests. Même s'il reste possible de réaliser un dispositif dans lequel le sens de traction peut être réglable, afin de faire varier l'angle de pelage, un tel dispositif sera beaucoup plus couteux et complexe à réaliser, alors que l'invention telle que décrite plus haute apporte déjà des réponses satisfaisantes aux problématiques de réglage de l'angle de pelage, via l'éprouvette précitée.

Dans un mode de réalisation préféré, le rouleau de front possède une surface cylindrique.

En position de tests, on pourra utiliser avantageusement une éprouvette de forme complexe, par exemple courbée variablement ou régulièrement sur son long, et/ou de faces opposées non parallèles, et/ou d'épaisseur variable, laquelle est en appui par sa surface encollée d'adhésif sur le rouleau de front, selon un plan tangent au rouleau de front qui forme un angle prédéfini avec le sens de traction, de préférence 90°. Cet angle de pelage est très largement utilisé pour les tests en pelage, et permet donc de comparer les tests effectués avec de nombreux autres tests.

Par ailleurs, lorsque l'éprouvette est directement issue d'aubes utilisées dans l'aéronautique par exemple, l'adhésif ne présente pas forcement une extrémité libre facilement préhensible par les moyens d'accrochage du dispositif de tests en pelage associé. En effet, une telle aube comprend une pale qui équivaut au support de l'éprouvette de tests, et un renfort qui équivaut à l'adhésif de l'éprouvette de tests. Le renfort recouvre en général les parties amont des surfaces d'extrados et d'intrados de la pale, ainsi que le bord d'attaque de la pale. Des coupes sont effectuées dans l'aube renforcée afin d'en extraire ces éprouvettes qui permettent de tester le maintien du renfort sur la pale. En particulier, il est intéressant de tester la résistance au décollage du renfort à partir du bord d'attaque de la pale, car c'est le cas de figure le plus susceptible de survenir lors d'une utilisation fonctionnelle et réelle de l'aube, par exemple lors d'une collision avec un objet provenant de l'amont de l'aube. Comme le renfort ne présente pas de partie préhensible au niveau du bord d'attaque de la pale, et comme il est à la fois collé sur les surfaces d'extrados et d'intrados de la pale, il est actuellement impossible d'effectuer un test de pelage exploitable.

C'est aussi pourquoi l'invention propose ladite éprouvette

On insistera si nécessaire sur le fait que l'éprouvette définie ci-dessus doit être considérée comme une invention en elle-même, car elle apporte une solution exhaustive au problème de pelage d'éprouvettes issues d'aubes.

Il a été trouvé ici une solution permettant d'effectuer des tests en pelage utiles sur des éprouvettes issues d'aubes, puisque, grâce à la fente, on a séparé le renfort en deux plaques distinctes, l'une collée sur la surface d'intrados de la pale, et l'autre collée sur la surface d'extrados de la pale. Chacune de ces plaques conserve toutefois une portion s'étendant au-delà du bord d'attaque de la pale. Cette même portion a été préparée de manière à ce qu'elle comprenne des moyens d'attache adaptés aux moyens d'accrochage du dispositif de tests en pelage.

On notera aussi qu'une telle éprouvette est tout à fait adaptée à des tests en pelage sélectifs, puisqu'elle pourra présenter les particularités héritées de l'aube à partir de laquelle on l'aura formée : forme complexe, avec des faces non parallèles (les surfaces d'intrados et d'extrados) ainsi qu'une épaisseur et une courbure propres et potentiellement variables.

Avantageusement, les moyens d'attache pourront comprendre des encoches formées sur des rebords opposés de la plaque. Ces moyens d'attache seront favorablement adaptés au câble du dispositif de tests en pelage décrit plus haut : le câble pourra être bouclé autour de la portion de plaque dépassant du bord d'attaque de la pale, tout en passant dans les encoches pour assurer une prise fiable. Cette réalisation est particulièrement intéressante car elle permettra au câble d'avoir une prise bien répartie sur la plaque.

On pourra, de manière similaire, percer la plaque d'un trou destiné à accueillir une extrémité d'un câble. Dans ce cas, le câble pourra par exemple être sécurisé dans sa position par un verrou épais fixé à son extrémité, et ne pourra donc pas retraverser le trou de la plaque.

La pale comprendra par exemple un composite à matrice organique, et le renfort comprendra par exemple du titane. Ceci correspond aux cas de figure les plus susceptibles d'être rencontrés.

Préférentiellement, les faces d'intrados et d'extrados ne sont pas parallèles, l'épaisseur entre ces faces et leurs courbures respectives étant en outre variables le long de la pale.

Comme déjà évoqué plus haut, de telles éprouvettes sont directement issues d'aubes utilisées dans l'aéronautique, et permettent donc de simuler au mieux leurs comportements.

L'invention concerne par ailleurs un procédé de fabrication d'une éprouvette telle que décrite précédemment, comprenant les étapes consistant à
- fournir une aube comprenant une pale qui comporte une surface d'intrados, une surface d'extrados, un bord d'attaque et un bord de fuite, et comprenant un renfort d'une seule pièce qui recouvre et est collé sur au moins une partie de la surface d'intrados, une partie de la surface d'extrados, et la totalité du bord d'attaque ou du bord de fuite,
- prélever une portion de cette aube, la portion comprenant au moins une partie du bord d'attaque ou du bord de fuite de la pale recouverte du renfort,
- fendre le renfort sur toute la longueur du bord d'attaque ou du bord de fuite, de manière à ce que le renfort soit séparé en deux plaques distinctes l'une de l'autre se faisant face de part et d'autre de la fente au-delà du bord d'attaque ou du bord de fuite de la pale, et
- former sur au moins une des plaques, au-delà du bord d'attaque ou du bord de fuite de la pale, des moyens d'attache procurant une prise à cette même plaque.

Les moyens d'attache pourront donc consister en des encoches formées sur des bords opposés de la plaque.

Différents aspects de l'invention pourront être mieux compris et d'autres détails, caractéristiques et avantages de la présente invention apparaîtront plus clairement à la lecture de la description qui suit, faite à titre d'exemple non limitatif et en référence aux dessins annexés, dans lesquels :
- la figure 1, déjà décrite, est une vue schématique d'un dispositif de tests en pelage selon les propositions de la norme NF EN 1464 ;
- les figures 2 et 3 sont des vues de profil et de face d'une éprouvette de tests en pelage issue d'une aube, selon l'invention ;
- la figure 4 est un schéma en perspective d'un dispositif de tests en pelage, avec une éprouvette selon l'invention.

Selon les figures 2 et 3, on voit une éprouvette 40 issue d'une aube qui a été coupée selon des plans perpendiculaires à son bord d'attaque. Une telle aube comprend une pale 42, en matériau composite à matrice organique, qui constitue le support de l'éprouvette de l'invention. Elle comprend aussi un renfort 44, en titane, qui recouvre la partie amont de l'aube, et qui constitue l'adhésif de l'éprouvette de l'invention. Dans la suite, on se référera à l'éprouvette, en continuant à utiliser la notion de pale pour désigner le support, et la notion de renfort pour désigner l'adhésif. Comme on peut le voir, la pale présente une surface d'intrados 42a, une surface d'extrados 42b et un bord d'attaque 46, qui est dépassé par le renfort 44. Le bord d'attaque 46 est métallique. Il peut être en titane. Son épaisseur e suivant l'axe général 40a d'allongement de l'éprouvette 40 sera typiquement comprise entre 50 et 100mm. Les surfaces d'intrados et d'extrados de l'éprouvette encollées du renfort 44 sont courbées différemment l'une de l'autre, et l'épaisseur de la pale 42 varie entre ces surfaces. Bien que le renfort 44 soit à l'origine formé d'une seule pièce recouvrant la totalité du bord d'attaque 46 de la pale, ainsi qu'une partie de ses surfaces d'intrados et d'extrados, le renfort comprend ici une fente traversante 48 mettant à jour la totalité du bord d'attaque 46 de la pale de l'éprouvette. De cette manière, le renfort 44 est séparé en deux plaques 44a et 44b distinctes se faisant face de part et d'autre de la fente 48 au-delà du bord d'attaque 46. Le renfort 44 s'étend cependant toujours sur toute la largeur de l'éprouvette, même s'il n'est pas présent jusqu'au bord de fuite de la pale 42.

Les plaques 44a et 44b comprennent, entre le bord d'attaque 48 de la pale et son extrémité, des encoches 50 opposées et sensiblement symétriques usinées sur leurs bords latéraux. Ces encoches 50 servent de prises à un câble bouclé que l'on pourra passer dans la fente 48 entre les plaques 44a et 44b, puis engager dans les encoches 50. Alternativement, les plaques 44a, 44b, comprennent au-delà du bord d'attaque 46 des trous 52 respectifs qui pourront aussi servir à assurer une prise ; par exemple en y insérant et bloquant l'extrémité d'un câble.

L'aube ainsi coupée et préparée devient une éprouvette exploitable pour des tests en pelage, car elle comprend un adhésif distinct sur chacune de ses faces, incarné par chaque plaque 44a, 44b, dont une extrémité est dégagée du support, incarné par la pale 42, et présente des moyens d'attache 50, 52 aisément préhensibles.

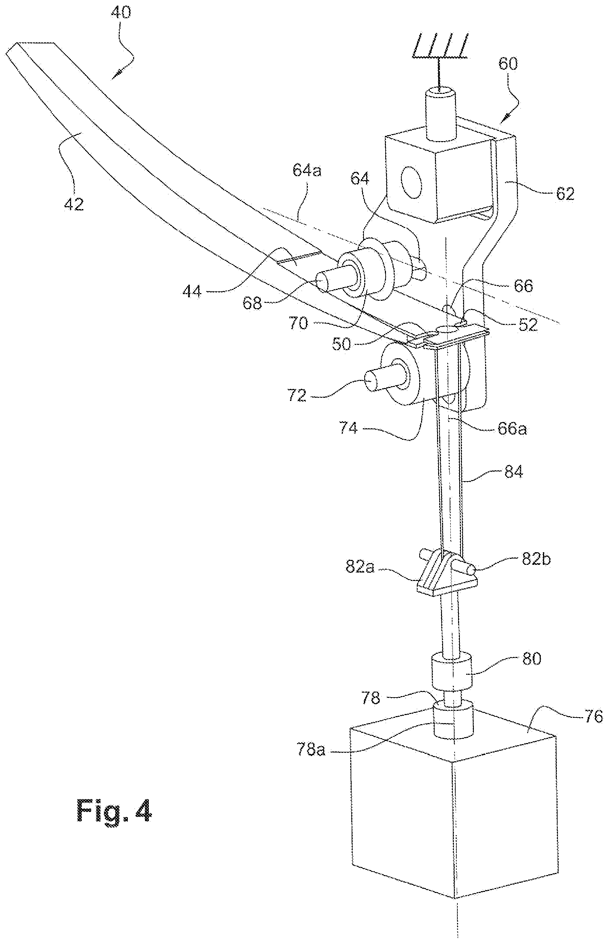

En référence à la figure 4, on décrit maintenant un exemple de dispositif de tests en pelage utilisant une éprouvette selon l'invention, et comprenant ici l'éprouvette représentée aux figures 2 et 3. Comme expliqué plus avant, ce dispositif de test en pelage peut être utilisé avec une large gamme d'éprouvettes de formes complexes, ou simple, autres que celle ici représentée.

Un bâti 60 est fixé. Ce bâti comprend un mur 62 vertical comprenant deux lumières 64, 66, l'un 64 de forme oblongue avec un grand axe horizontal 64a, et l'autre 66 sensiblement isométrique de grand axe vertical 66a. Les dimensions des deux lumières peuvent toutefois être complètement différentes.

La lumière 64 sert de passage à un arbre 68 perpendiculaire au mur 62, de diamètre correspondant au petit axe de la lumière 64. Cet arbre 68 est réglable en translation dans la lumière 64 dans la direction horizontale du grand axe 64a. Un rouleau de guidage 70 creux est monté sur l'arbre 68 d'un côté du mur 62. Ce rouleau est libre en rotation autour de l'arbre 68. Le rouleau de guidage 70 possède une surface locale annulaire d'appui externe d'aspect linéaire, comme il a été détaillé plus haut dans ce document. Bien que représenté dans la figure comme possédant une surface torique, le rouleau 70 pourrait donc de manière équivalente posséder une surface complètement sphérique par exemple.

La lumière 66 sert de passage à un arbre 72 perpendiculaire au mur 62, de diamètre correspondant au petit axe de la lumière 66. Cet arbre 72 est réglable en translation dans la lumière 66 dans la direction verticale du grand axe 66a. Un rouleau de front 74 creux est monté sur l'arbre 72 du même côté du mur 62 que le rouleau de guidage 70. Ce rouleau de front est libre en rotation autour de l'arbre 72. Le rouleau de front 74 possède une surface cylindrique.

Le rouleau de guidage 70 est, dans toute sa plage de réglage, en translation, décalée horizontalement et verticalement du rouleau de front 74.

Un deuxième mur, non représenté sur les figures, est classiquement fixé en face du premier mur 62 de l'autre côté des rouleaux. Ce deuxième mur comprend des lumières symétriques aux lumières 64, 66 du premier mur 62, dans lesquelles les arbres 68, 72 peuvent prendre en deuxième appui de l'autre côté des rouleaux. Les charges sont ainsi réparties égalitairement sur les arbres de part et d'autres des rouleaux 70, 74, qui subissent des contraintes transversales en fonctionnement. Ceci évite la déformation des arbres.

On trouve sous le bâti 60 des moyens de traction 76 comprenant un vérin 78 vertical, d'axe 78a vertical tangent à la surface du rouleau de front 74, du côté du rouleau de front opposé au rouleau de guidage 70. Un capteur de force 80 est intégré au vérin 78, et mesure la force de traction vertical exercée par le vérin. On notera que le capteur de force peut de manière équivalente être monté n'importe le long de la chaîne de traction. A l'extrémité supérieure du vérin 78 est agencé un ensemble chape 82a et axe 82b. Dans la chape 82a et autour de l'axe 82b est montée une boucle 84 de câble, par exemple en kevlar™, c'est-à-dire en poly(p-phénylènetéréphtalamide).

On a agencé l'éprouvette 40 décrite en référence aux figures 3 et 4 entre le rouleau de guidage 70 et le rouleau de front 74. L'extrémité fendue du renfort 44 dépasse légèrement du rouleau de front 74. La boucle 84 est engagée dans la fente 48 de l'éprouvette et dans les encoches 50, de sorte qu'une des plaques 44b est en prise avec la boucle 84.

La boucle 84 est mise en tension par le vérin 78, et appuie la face inférieure de l'éprouvette 40 contre le rouleau de front 74. L'éprouvette tend alors à pivoter autour du rouleau de front 74, et se plaque par sa face supérieure sur le rouleau de guidage 70. On voit donc bien qu'on fonction de la position relative des deux rouleaux, l'éprouvette n'aura pas la même orientation. En revanche, quel que soit la position du rouleau de front 74 dans la lumière verticale 66, la boucle 84 sera toujours en tension verticalement et tangentiellement au rouleau de front 74.

En fonctionnement, la boucle 84 tire la plaque 44b vers le bas et la décolle de la pale 42. L'éprouvette 40 est libre de se déplacer par roulement le long des rouleaux 70, 74, de sorte que le front de roulage se situe toujours sensiblement à la verticale de la boucle 84. Le test de pelage s'effectue en relevant les données délivrées par le capteur de force 80 alors que le vérin 78 se déplace vers le bas à vitesse constante.

On peut aisément automatiser le déplacement en translation des rouleaux 70, 74 dans les lumières 64, 66, pendant le déplacement du vérin 78, afin qu'en tout temps, selon les variations de courbure et d'épaisseur de l'éprouvette 40, l'orientation de l'éprouvette tangentiellement au rouleau de front 74 soit conservée.

On pourra bien évidemment modifier le système d'attache composé des encoches 50, de la boucle 84, de la chape 82a et de l'axe 82b, par tout autre système d'attache connu et adapté à saisir efficacement l'adhésif de l'éprouvette et à l'entrainer vers le bas.

## Revendications

1. Eprouvette (40) de tests en pelage, **caractérisée en ce que** :
- l'éprouvette est issue d'une aube, et comprend :
-- une portion de pale (42) de l'aube qui comporte une surface d'intrados (42a), une surface d'extrados (42b), et un bord d'attaque (46) et/ou un bord de fuite, et
-- un renfort de l'aube (44) qui recouvre et est collé sur au moins une partie de la surface d'intrados et une partie de la surface d'extrados, et qui s'étend au-delà de l'aube et du bord d'attaque et/ou du bord de fuite de la portion de pale,
- ledit renfort (44) est fendu sur toute la longueur du bord d'attaque (46) et/ou du bord de fuite, de manière à ce que le renfort soit séparé en deux plaques (44a, 44b) distinctes l'une de l'autre se faisant face de part et d'autre de la fente (48) au-delà du bord d'attaque et/ou du bord de fuite de la portion de pale, et
- au moins une des plaques comprend en outre, au-delà du bord d'attaque et/ou du bord de fuite de la portion de pale, des moyens d'attache (50, 52) procurant à cette même plaque une prise pour une traction.

2. Eprouvette selon la revendication 1, **caractérisée en ce que** les moyens d'attache comprennent des encoches (50) formées sur des rebords opposés de la plaque.

3. Eprouvette selon l'une des revendications 1 ou 2, **caractérisée en ce que** les faces d'intrados (42a) et d'extrados (42b) ne sont pas parallèles, l'épaisseur entre ces faces et leurs courbures respectives étant en outre variables le long de la pale (42).

4. Eprouvette selon l'une des revendications 1 à 3, **caractérisée en ce que** la pale (42) comprend un composite à matrice organique, et le renfort (44) comprend du titane.

5. Eprouvette selon l'une des revendications 1 à 4, **caractérisée en ce qu'**elle est issue d'une aube de turbomachine.

6. Procédé de fabrication d'une éprouvette (40) selon l'une des revendications 1 à 5, comprenant les étapes consistant à :
- fournir une aube comprenant :
-- une pale (42) qui comporte une surface d'intrados (42a), une surface d'extrados (42b), un bord d'attaque (46) et un bord de fuite, et
-- un renfort (44) d'une seule pièce qui recouvre et est collé sur au moins une partie de la surface d'intrados, une partie de la surface d'extrados, et la totalité du bord d'attaque ou du bord de fuite,
- prélever une portion de cette aube, la portion comprenant au moins une partie du bord d'attaque (46) ou du bord de fuite de la pale recouverte du renfort,
- fendre le renfort (44) sur toute la longueur du bord d'attaque ou du bord de fuite, de manière à ce que le renfort soit séparé en deux plaques (44a, 44b) distinctes l'une de l'autre se faisant face de part et d'autre de la fente (48) au-delà du bord d'attaque ou du bord de fuite de la pale, et
- former sur au moins une des plaques, au-delà du bord d'attaque ou du bord de fuite de la pale, des moyens d'attache (50, 52) procurant une prise à cette même plaque pour des moyens d'accrochage, à fin de traction.

## Patentansprüche

1. Schältest-Prüfkörper (40), **dadurch gekennzeichnet, dass**
- der Prüfkörper aus einer Schaufel stammt und enthält:
-- einen Schaufelblattabschnitt (42), der eine Saugseite (42a), eine Druckseite (42b) und eine Anströmkante (46) und/oder eine Abströmkante aufweist, und
-- eine Schaufelverstärkung (44), die zumindest einen Teil der Druckseite und einen Teil der Saugseite bedeckt und damit verklebt ist und die sich über die Schaufel und die Anströmkante und/oder Abströmkante des Schaufelblattabschnitts hinaus erstreckt,
- wobei die Verstärkung (44) über die gesamte Länge der Anströmkante (46) und/oder der Abströmkante geschlitzt ist, so dass die Verstärkung in zwei getrennte Platten (44a, 44b) aufgeteilt ist, die beiderseits des Schlitzes (48) über die Anströmkante und/oder die Abströmkante des Schaufelblattabschnitts hinaus einander gegenüberliegen, und
- wobei zumindest eine der Platten ferner über die Anströmkante und/oder die Abströmkante des Schaufelblattabschnitts hinaus Befestigungsmittel (50, 52) aufweist, die an derselben Platte eine Angriffsstelle zum Ziehen schaffen.

2. Prüfkörper nach Anspruch 1,
**dadurch gekennzeichnet, dass**
die Befestigungsmittel Kerben (50) aufweisen, die an entgegengesetzten Randleisten der Platte ausgebildet sind.

3. Prüfkörper nach einem der Ansprüche 1 oder 2,
**dadurch gekennzeichnet, dass**
die Druckseite (42a) und die Saugseite (42b) nicht parallel verlaufen, wobei die Dicke zwischen diesen Seiten und ihre jeweiligen Krümmungen ferner entlang des Schaufelblatts (42) variabel sind.

4. Prüfkörper nach einem der Ansprüche 1 bis 3,
**dadurch gekennzeichnet, dass**
das Schaufelblatt (42) einen Verbundstoff mit organischer Matrix enthält und die Verstärkung (44) Titan enthält.

5. Prüfkörper nach einem der Ansprüche 1 bis 4,
**dadurch gekennzeichnet, dass**
er aus einer Schaufel einer Turbomaschine bzw. eines Turbotriebwerks stammt.

6. Verfahren zum Herstellen eines Prüfkörpers (40) nach einem der Ansprüche 1 bis 5, umfassend die Schritte:
- Bereitstellen einer Schaufel, enthaltend
-- ein Schaufelblatt (42), das eine Saugseite (42a), eine Druckseite (42b), eine Anströmkante (46) und eine Abströmkante aufweist, und
-- eine einstückig ausgeführte Verstärkung (44), die zumindest einen Teil der Druckseite, einen Teil der Saugseite und die gesamte Anströmkante oder Abströmkante bedeckt und damit verklebt ist,
- Entnehmen eines Abschnitts dieser Schaufel, wobei der Abschnitt zumindest einen Teil der Anströmkante (46) oder der Abströmkante des mit der Verstärkung bedeckten Schaufelblatts enthält,
- Schlitzen der Verstärkung (44) über die gesamte Länge der Anströmkante oder der Abströmkante, so dass die Verstärkung in zwei getrennte Platten (44a, 44b) aufgeteilt ist, die beiderseits des Schlitzes (48) über die Anströmkante oder die Abströmkante des Schaufelblatts hinaus einander gegenüberliegen, und
- Ausbilden von Befestigungsmitteln (50, 52) an zumindest einer der Platten über die Anströmkante oder die Abströmkante des Schaufelblatts hinaus, die eine Angriffsstelle an derselben Platte für Einhakmittel zum Ziehen schaffen.

## Claims

1. Coupon (40) suitable for peeling tests, **characterised in that** the coupon is derived from a vane and comprises :
- a blade portion (42) of the vane that comprises an intrados side surface (42a), an extrados side surface (42b) and a leading edge (46) and/or a trailing edge and
- a vane reinforcement (44) that covers and is glued to at least a part of the intrados side surface and a part of the extrados side surface, and which extends beyond the vane and beyond the leading and/or trailing edge of the blade portion,
wherein the reinforcement (44) is split over the entire length of the leading edge (46) and/or trailing edge, such that the reinforcement is separated into two plates (44a, 44b) separate from one another and facing each other on either side of the slit (48) beyond the leading edge and/or trailing edge of the blade portion,
and wherein at least one of the plates furthermore comprises, beyond the leading edge and/or trailing edge of the blade portion, fastening means (50, 52) providing a hold to said plate for traction.

2. Coupon according to claim 1, **characterised in that** the fastening means comprise notches (50) formed on opposite edges of the plate.

3. Coupon according to either of claims 1 or 2, **characterised in that** the intrados side (42a) and the extrados side (42b) surfaces are not parallel, with a thickness between said surfaces and their respective curvatures being furthermore variable along the blade (42).

4. Coupon according to any of claims 1 to 3, **characterised in that** the blade (42) comprises an organic matrix composite and the reinforcement (44) comprises titanium.

5. Coupon according to any of claims 1 to 4, **characterised in that** it is derived from a turbine engine vane.

6. A method of manufacturing a coupon (40) according to any of claims 1 to 5, comprising the stages involving:
- supplying a vane comprising:
-- a blade (42) that comprises an intrados side surface (42a), an extrados side surface (42b), a leading edge (46) and a trailing edge and
-- a reinforcement (44) formed of a single piece that covers and is glued to at least a part of the intrados side surface, a part of the extrados side surface and the entire leading and/or trailing edge,
- sampling a portion of said vane, wherein the portion comprises at least a part of the leading edge (46) or trailing edge of the blade covered with the reinforcement,
- splitting the reinforcement (44) over the entire length of the leading or trailing edge such that the reinforcement is separated into two plates (44a, 44b) separate from one another and facing each other on either side of the slit (48) beyond the leading or trailing edge of the blade and
- forming on at least one of the plates, beyond the leading or trailing edge of the blade, fastening means (50, 52) providing a hold to said plate for means of attachment, for the purpose of traction.
